# FASCICULE DE BREVET EUROPEEN

(11) **EP 3 292 400 B1**
(45) Date de publication et mention de la délivrance du brevet: **20.03.2019**
(21) Numéro de dépôt: 16727309.3
(22) Date de dépôt: 04.05.2016
(51) Int. Cl.: G01N 27/62, G01N 33/68, H01J 49/00, H01J 49/04, H01J 49/42

(54) **PROCEDE DE CARACTERISATION D'IONS**
VERFAHREN ZUR CHARAKTERISIERUNG VON IONEN
METHOD FOR CHARACTERISING IONS

(30) Priorité: 07.05.2015 FR 1554093
(43) Date de publication de la demande: 14.03.2018
(73) Titulaire: Aix-Marseille Université, 13284 Marseille Cedex 07 (FR); Centre National de la Recherche Scientifique CNRS, 75794 Paris Cedex 16 (FR)
(72) Inventeur: FERAUD, Géraldine, 06140 Vence (FR); JOUVET, Christophe, 13013 Marseille (FR); DEDONDER, Claude, 13013 Marseille (FR)
(74) Mandataire: Cabinet Camus Lebkiri
(86) Numéro de dépôt international: PCT/EP2016/060057
(87) Numéro de publication internationale: WO 2016/177812

(56) Documents cités:
- WO-A2-2013/005060
- CLAUDE DEDONDER ET AL: "Communication: Identification of daughter ions through their electronic spectroscopy at low temperature", JOURNAL OF CHEMICAL PHYSICS, vol. 141, no. 13, 131101, 7 octobre 2014 (2014-10-07), pages 1-5, XP055263199, US ISSN: 0021-9606, DOI: 10.1063/1.4896981 cité dans la demande
- HYUK KANG ET AL: "New Method for Double-Resonance Spectroscopy in a Cold Quadrupole Ion Trap and Its Application to UV-UV Hole-Burning Spectroscopy of Protonated Adenine Dimer", JOURNAL OF PHYSICAL CHEMISTRY LETTERS, vol. 5, no. 15, 7 août 2014 (2014-08-07), pages 2760-2764, XP055263321, US ISSN: 1948-7185, DOI: 10.1021/jz5012466
- OLEG V. BOYARKIN ET AL: "Electronic Spectroscopy of Cold, Protonated Tryptophan and Tyrosine", JOURNAL OF THE AMERICAN CHEMICAL SOCIETY, vol. 128, no. 9, 8 février 2006 (2006-02-08), pages 2816-2817, XP055263350, US ISSN: 0002-7863, DOI: 10.1021/ja058383u

## Description

### DOMAINE TECHNIQUE DE L'INVENTION

Le domaine technique de l'invention est celui de la caractérisation d'ions. La présente invention concerne un procédé de caractérisation d'ions. On décrit aussi un dispositif de caractérisation d'ions pour la mise en oeuvre d'un procédé de caractérisation d'ions selon l'invention.

La présente invention trouve notamment des applications dans les domaines de la chimie analytique, de la pharmacologie et de l'environnement.

### ARRIERE-PLAN TECHNOLOGIQUE DE L'INVENTION

Le domaine de la spectrométrie de masse est actuellement en pleine expansion. De nombreux industriels proposent des instruments, notamment utilisés dans les domaines de la chimie analytique, de la pharmacologie et de l'environnement, afin de caractériser des systèmes. Cependant, ces instruments permettent uniquement de caractériser le rapport masse/charge, noté m/z, des systèmes détectés, et non pas, notamment, les différents isomères ou tautomères des systèmes détectés.

La spectrométrie de masse en tandem (« tandem mass spectrometry » en anglais), notée MS/MS ou MS², est une technique de spectrométrie de masse pour l'identification et la caractérisation d'ions. La technique MS/MS consiste à :
- isoler dans un spectromètre de masse des ions de première génération, ayant un rapport masse/charge particulier ;
- activer les ions parents, par exemple par une technique de dissociation induite par collision CID (de l'anglais « Collision Induced Dissociation ») ;
- isoler un fragment particulier issu de l'activation des ions de première génération ;
- activer ledit fragment particulier, par exemple par la technique CID, afin d'obtenir des fragments de troisième génération.

L'étude des ions de troisième génération permet d'obtenir des informations sur les ions de deuxième génération, et permet donc indirectement d'obtenir des informations sur les ions de première génération. L'étude des ions de troisième génération peut par exemple être réalisée au moyen d'une spectroscopie électronique.

L'excitation optique, ou photo-fragmentation, est une autre technique de fragmentation d'ions qui peut être utilisée. On utilise typiquement :
- soit une technique de photo-fragmentation avec des photons IR pour tous types d'ions,
- soit une technique de photo-fragmentation visible ou UV pour des ions ayant un chromophore.

La spectroscopie électronique d'un ion permet d'obtenir un spectre donnant une information sur la structure globale de cet ion, mais il demeure difficile de déterminer la forme tautomère de l'ion, notamment en raison d'un phénomène de congestion spectrale lorsque l'ion est chaud. Le phénomène de congestion spectrale traduit le fait que le spectre obtenu, qui contient des raies fines caractéristiques lorsque l'ion est froid, devient une bande large et non caractéristique lorsque l'ion est chaud. On note qu'un ion à température ambiante est typiquement un ion chaud.

Les inventeurs sont auteurs de l'article « Communication : Identification of daughter ions through their electronic spectroscopy at low temperature », The Journal of Chemical Physics (2014). Cet article propose une méthode de caractérisation de la structure isomère d'un photo-produit ionique, selon laquelle :
- des ions de première génération sont piégés dans un piège ionique ;
- les ions de première génération piégés sont refroidis ;
- les ions de première génération refroidis sont photo-fragmentés : des ions de deuxième génération sont obtenus ;
- les ions de deuxième génération sont refroidis ;
- les ions de deuxième génération refroidis sont photo-fragmentés : des ions de troisième génération sont obtenus ;
- les ions de troisième génération sont détectés.

Dans l'article, la méthode de caractérisation est appliquée au cas particulier de la tyrosine protonée (« protonated tyrosine » en anglais) TyrH⁺ et permet de caractériser structurellement les différents photo-produits de TyrH⁺. La méthode ayant permis de caractériser les fragments issus d'un acide aminé protoné, il est envisagé en conclusion de l'article de tester la méthode pour caractériser les fragments issus d'un peptide protoné, d'une manière plus complète que la caractérisation obtenue par spectrométrie de masse.

Toutefois, les expériences menées afin d'appliquer ladite méthode à d'autres ions que la tyrosine protonée TyrH⁺ ne sont pas concluantes.

Dans un autre article de l'inventeur, << New Method for Double-Resonance Spectroscopy in a Cold Quadrupole Ion Trap and Its Application to UV-UV Hole-Burning Spectroscopy of Protonated Adenine Dimer >>, The Journal of Physical Chemistry Letters (2014), les dimères protonés d'adénine Ade₂H⁺ sont analysés par spectroscopie à double résonance dans un piège ionique froid quadripolaire.

### RESUME DE L'INVENTION

L'invention offre une solution aux problèmes évoqués précédemment, en proposant un procédé de caractérisation d'ions, et en particulier un procédé de caractérisation de la structure isomère d'ions, le procédé étant applicable à tous les types d'ions.

L'invention concerne donc un procédé de caractérisation d'ions comportant :
- une étape selon laquelle une pluralité d'ions de première génération est piégée dans un piège ionique ;
- une étape selon laquelle la pluralité d'ions de première génération piégée dans le piège ionique est refroidie ;
- une étape selon laquelle la pluralité d'ions de première génération refroidis est photo-fragmentée pour l'obtention d'une pluralité d'ions de deuxième génération, la pluralité d'ions de deuxième génération étant différente de la pluralité d'ions de première génération, la pluralité d'ions de deuxième génération étant au moins d'un premier type ;
- une étape i) selon laquelle le premier type d'ions de deuxième génération est sélectionné dans le piège ionique en éjectant hors du piège ionique tout ion de première génération résiduel et tout ion de deuxième génération d'un type différent du premier type ;
- une étape ii) selon laquelle les ions de deuxième génération du premier type sélectionnés et piégés dans le piège ionique sont refroidis ;
- une étape iii) selon laquelle les ions de deuxième génération du premier type refroidis sont photo-fragmentés pour l'obtention d'une pluralité d'ions de troisième génération, la pluralité d'ions de troisième génération étant différente de la pluralité d'ions de deuxième génération, la pluralité d'ions de troisième génération étant au moins d'un premier type ;
- une étape selon laquelle la pluralité d'ions de dernière génération est détectée.

La première photo-fragmentation de la pluralité d'ions de première génération permet d'obtenir la pluralité d'ions de deuxième génération. Les ions de deuxième génération sont des photo-fragments des ions de première génération, donc les ions de deuxième génération sont différents des ions de première génération. Les ions de deuxième génération peuvent être tous d'un même type, ou les ions de deuxième génération peuvent être de types différents. On entend par « ions de types différents » des ions pouvant être sélectionnés indépendamment les uns des autres. En outre, des ions de première génération peuvent subsister à l'issue de la première photo-fragmentation. L'étape i) permet d'isoler un premier type d'ions de deuxième génération dans le piège ionique. Ainsi, on garantit que la deuxième photo-fragmentation est réalisée uniquement sur ce premier type d'ions de deuxième génération. Les ions de troisième génération sont donc des photo-fragments de ce premier type d'ions de deuxième génération. Isoler le premier type d'ions de deuxième génération dans le piège ionique avant de réaliser la deuxième photo-fragmentation permet une caractérisation fiable du premier type d'ions de deuxième génération, en utilisant les ions de troisième génération obtenus. En effet, la signature spectroscopique du premier type d'ions de deuxième génération est obtenue par la détection de ses photo-fragments, c'est-à-dire par la détection des ions de troisième génération. Or des ions identiques aux ions de troisième génération peuvent également être obtenus, notamment, par photo-fragmentation d'ions de première génération résiduels ou par photo-fragmentation d'ions de deuxième génération d'un type différent du premier type. Il est également possible que des ions de générations différentes absorbent dans le même domaine spectral. Par exemple, un ion de première génération et un ion de deuxième génération peuvent absorber tous les deux dans l'UV. Dans de tels cas, la caractérisation fiable du premier type d'ions de deuxième génération est rendue impossible car la signature spectroscopique du premier type d'ions de deuxième génération est mélangée à la signature spectroscopique d'autres ions tels que des ions de première génération et/ou des ions de deuxième génération d'un type différent du premier type. Ainsi, la méthode décrite dans la publication précédemment citée fonctionne lorsqu'elle est appliquée au cas particulier de la tyrosine protonée TyrH⁺ car la tyrosine protonée TyrH⁺ absorbe dans l'UV tandis que les ions de deuxième génération - c'est-à-dire, les photo-fragments de la tyrosine protonée TyrH⁺, absorbent dans le visible.

Plus généralement, isoler un unique type d'ions de Nième génération avant de réaliser une Nième photo-fragmentation permet une caractérisation fiable du type d'ions de Nième génération.

Outre les caractéristiques qui viennent d'être évoquées dans le paragraphe précédent, le procédé de caractérisation selon l'invention peut présenter une ou plusieurs caractéristiques complémentaires parmi les suivantes, considérées individuellement ou selon toutes les combinaisons techniquement possibles :
- Le procédé comporte une étape selon laquelle la pluralité d'ions de première génération est sélectionnée par une technique de spectrométrie de masse.
- L'étape selon laquelle la pluralité d'ions de dernière génération est détectée est réalisée par une technique de spectrométrie de masse.
- Les étapes i), ii) et iii) forment une séquence qui est réalisée N fois, N étant un entier naturel supérieur ou égal à 2, le numéro de chaque génération étant incrémenté de 1 à chaque réalisation de ladite séquence.

Il est également révélé, sans faire partie de l'invention, un dispositif de caractérisation d'ions pour la mise en oeuvre du procédé de caractérisation d'ions selon l'invention, le dispositif étant caractérisé en ce qu'il comporte :
- un piège ionique pour le piégeage d'une pluralité d'ions ;
- un module de refroidissement de la pluralité d'ions, comprenant une source de gaz tampon et un cryostat ;
- un laser de photo-fragmentation ;
- un spectromètre de masse pour la sélection d'un type d'ions d'intérêt ;
- un détecteur pour la détection d'une pluralité de photo-fragments.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.

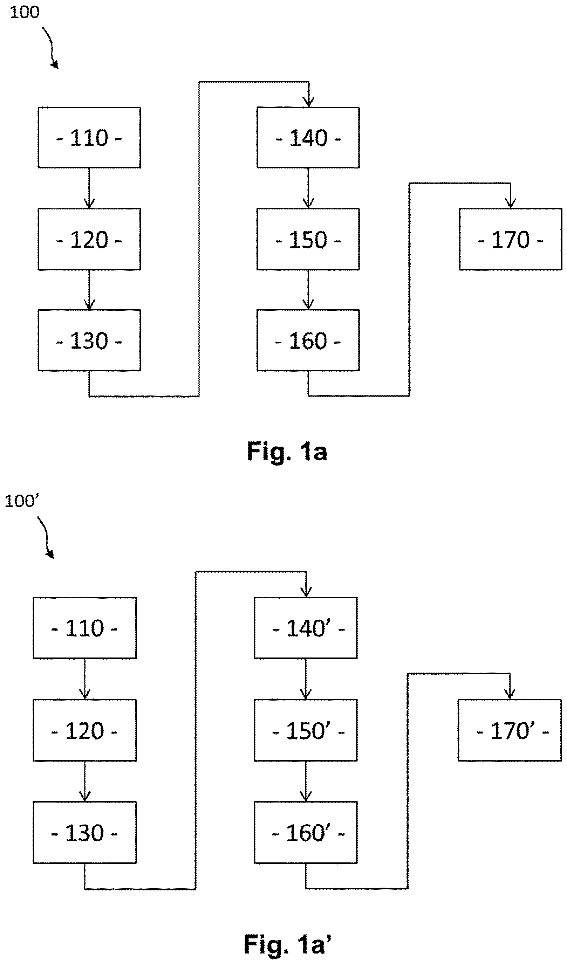

- La figure 1a montre un diagramme des étapes d'un procédé de caractérisation d'ions selon un premier mode de réalisation de l'invention.
- La figure 1a' montre un diagramme des étapes complémentaires du procédé de caractérisation d'ions selon une variante du premier mode de réalisation de l'invention.
- La figure 1b montre un diagramme des étapes d'un procédé de caractérisation d'ions selon un deuxième mode de réalisation de l'invention.
- La figure 2a montre schématiquement une première phase d'utilisation d'un dispositif de caractérisation d'ions pour la mise en oeuvre d'un procédé de caractérisation d'ions selon le premier ou le deuxième mode de réalisation de l'invention.
- La figure 2b montre schématiquement une deuxième phase d'utilisation d'un dispositif de caractérisation d'ions pour la mise en oeuvre d'un procédé de caractérisation d'ions selon le premier ou le deuxième mode de réalisation de l'invention.
- La figure 2c montre schématiquement une troisième phase d'utilisation d'un dispositif de caractérisation d'ions pour la mise en oeuvre d'un procédé de caractérisation d'ions selon le premier ou le deuxième mode de réalisation de l'invention.
- La figure 2d montre schématiquement une quatrième phase d'utilisation d'un dispositif de caractérisation d'ions pour la mise en oeuvre d'un procédé de caractérisation d'ions selon le premier mode de réalisation de l'invention.

### DESCRIPTION DETAILLEE D'AU MOINS UN MODE DE REALISATION DE L'INVENTION

Sauf précision contraire, un même élément apparaissant sur des figures différentes présente une référence unique.

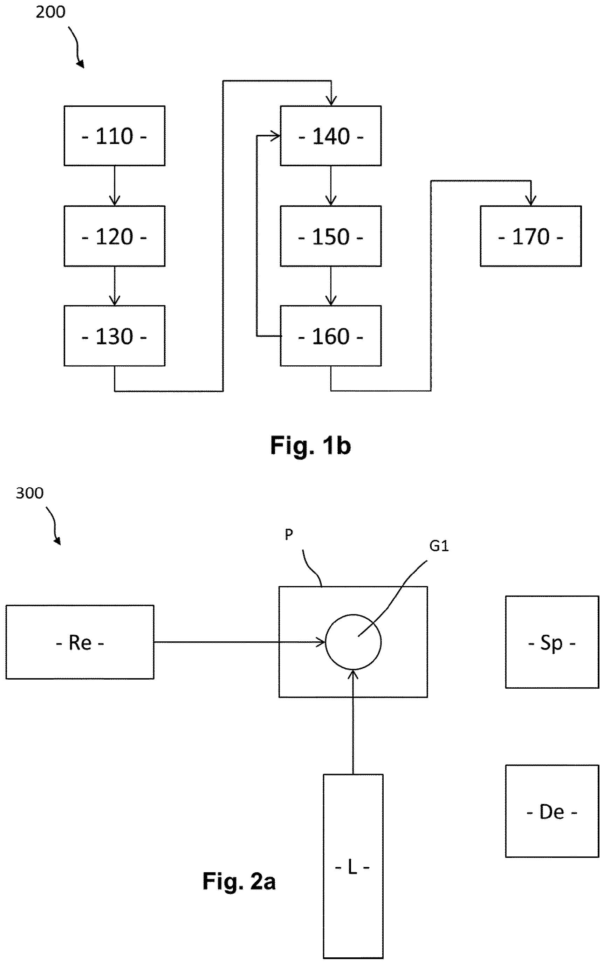

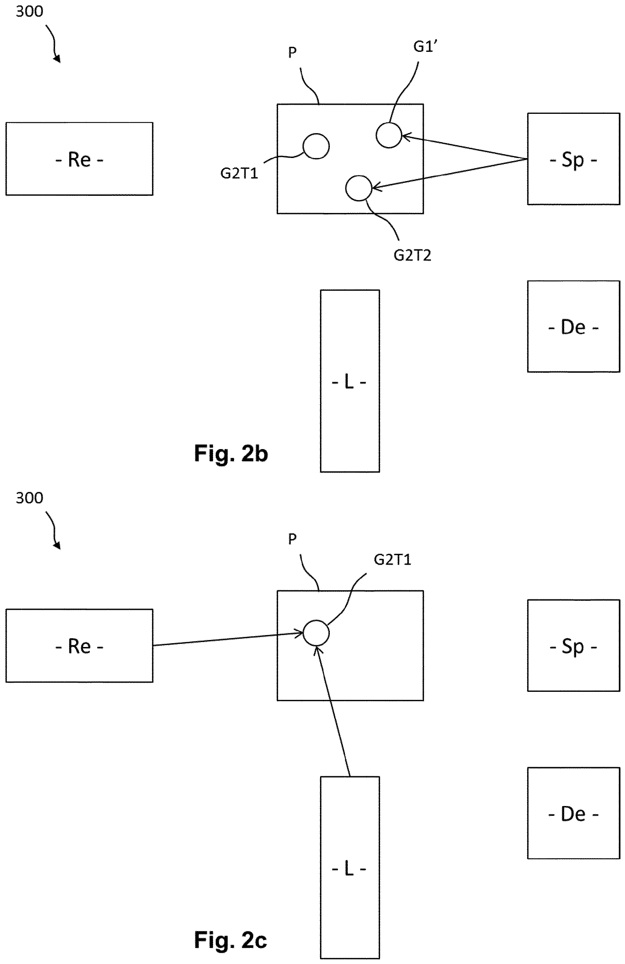

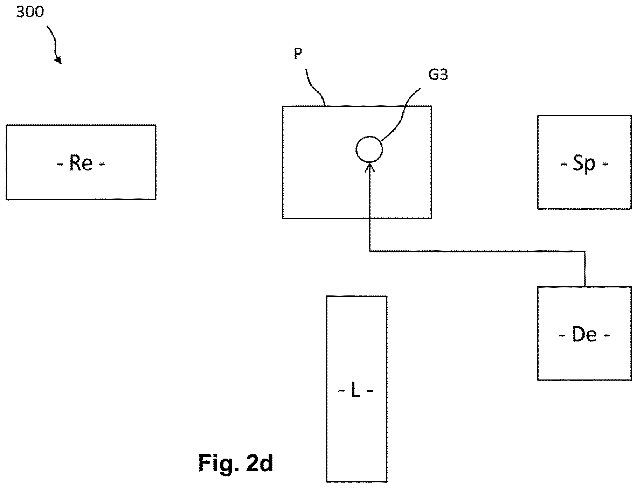

La figure 1a montre un diagramme des étapes d'un procédé 100 de caractérisation d'ions selon un premier mode de réalisation de l'invention. Les figures 2a, 2b, 2c et 2d montrent une utilisation d'un dispositif 300 de caractérisation d'ions pour la mise en oeuvre du procédé de caractérisation d'ions selon le premier mode de réalisation de l'invention. Les figures 1a et 2a à 2d sont décrites conjointement.

Les figures 1a et 2a montrent une étape 110, selon laquelle une pluralité G1 d'ions de première génération est piégée dans un piège ionique P. La pluralité G1 d'ions de première génération se présente sous la forme d'un nuage d'ions au sein du piège ionique P.

La pluralité G1 d'ions de première génération est typiquement obtenue en utilisant une technique d'ionisation par électronébulisation ESI (de l'anglais « electrospray ionization »).

Le piège ionique P est préférentiellement un piège quadripolaire, ou piège de Paul, qui permet une bonne localisation d'un nuage d'ions en son sein. Une bonne localisation du nuage d'ions au sein du piège ionique permet une interaction efficace entre un laser de photo-fragmentation et le nuage d'ions. Alternativement, le piège ionique P peut également être un piège de Penning, ou bien un piège linéaire multipolaire.

Le procédé 100 selon le premier mode de réalisation de l'invention peut avantageusement comporter une étape (non représentée) selon laquelle la pluralité G1 d'ions de première génération est sélectionnée de manière à obtenir une pluralité G1 d'ions de première génération d'un premier type. L'étape de sélection d'un premier type d'ions de première génération est par exemple réalisée par une technique de spectrométrie de masse. L'étape de sélection par spectrométrie de masse permet avantageusement d'obtenir une pluralité G1 d'ions de première génération ayant tous le même rapport masse/charge, noté m/z. L'étape de sélection par spectrométrie de masse peut par exemple avoir lieu avant que la pluralité G1 d'ions de première génération soit introduite et piégée dans le piège ionique P. Alternativement, l'étape de sélection par spectrométrie de masse peut également avoir lieu après que la pluralité G1 d'ions de première génération ait été introduite et piégée dans le piège ionique P. Dans ce dernier cas, l'étape de sélection consiste à conserver dans le piège ionique P les ions de première génération ayant le rapport m/z souhaité, en éjectant hors du piège ionique P les ions de première génération n'ayant pas le rapport m/z souhaité. L'étape de sélection d'un premier type d'ions de première génération peut également être réalisée par une technique de spectrométrie de mobilité ionique IMS (de l'anglais « ion mobility spectrometry »).
Les figures 1a et 2a montrent une étape 120 selon laquelle la pluralité G1 d'ions de première génération piégée dans le piège ionique P est refroidie au moyen d'un module de refroidissement Re. Le module de refroidissement Re comprend :
- une source de gaz tampon permettant d'introduire un gaz tampon dans le piège ionique P, et
- un cryostat.
Le gaz tampon est par exemple de l'hélium He. La source de gaz tampon peut donc être une bouteille d'hélium comprimé. Alternativement, la source de gaz tampon peut être une bouteille d'hélium à pression ambiante que l'on utilise avec un compresseur.

Les figures 1a, 2a et 2b montrent une étape 130 selon laquelle la pluralité G1 d'ions de première génération refroidis est photo-fragmentée au moyen d'un laser de photo-fragmentation L émettant à une première longueur d'onde λ1, pour l'obtention d'une pluralité d'ions de deuxième génération. La première longueur d'onde λ1 est choisie en fonction de la pluralité G1 d'ions de première génération à photo-fragmenter. La pluralité d'ions de deuxième génération est différente de la pluralité d'ions de première génération, et la pluralité d'ions de deuxième génération est au moins d'un premier type. A l'issue de la photo-fragmentation des ions de première génération, l'exemple particulier représenté aux figures 2a et 2b montre que le piège ionique contient :
- un premier type G2T1 d'ions de deuxième génération,
- un deuxième type G2T2 d'ions de deuxième génération, et
- des ions de première génération résiduels G1', qui n'ont pas été photo-fragmentés.
Naturellement, dans un autre exemple particulier (non illustré), le piège ionique P pourrait contenir des troisième, quatrième, ... nième types d'ions de deuxième génération à l'issue de la photo-fragmentation des ions de première génération. Le piège ionique P pourrait également ne contenir aucun ion de première génération résiduel, ou ne contenir qu'un seul type d'ions de deuxième génération.

Les figures 1a, 2b et 2c montrent une étape i), qui est référencée 140 en figure 1a, selon laquelle le premier type d'ions de deuxième génération G2T1 est sélectionné dans le piège ionique en éjectant hors du piège ionique tout ion de première génération résiduel et tout ion de deuxième génération d'un type différent du premier type. L'étape de sélection du premier type d'ions de deuxième génération G2T1 est réalisée en utilisant un spectromètre de masse Sp. Dans l'exemple particulier illustré aux figures 2b et 2c, le spectromètre de masse Sp permet l'éjection des ions de première génération résiduels G1' et du deuxième type d'ions de deuxième génération G2T2. Quel que soit le contenu du piège ionique P avant l'étape 140 de sélection, on garantit que le piège ionique P ne contient sensiblement plus que le premier type d'ions de deuxième génération G2T1 à l'issue de ladite étape 140 de sélection. On entend par « sensiblement » le fait qu'une petite quantité résiduelle ε des ions à éjecter peut subsister au sein du piège ionique P après l'étape 140 de sélection. La petite quantité résiduelle ε est suffisamment petite pour que son signal ne perturbe pas la mesure. La petite quantité résiduelle ε est préférentiellement inférieure à 10% de l'ensemble des ions à éjecter qui se trouvait dans le piège ionique P avant l'étape 140 de sélection.

La sélection se fait typiquement en ajoutant une tension radiofréquence sur une électrode du piège ionique P. Cette tension radiofréquence est choisie et ajustée pour éjecter les ions ayant un certain rapport m/z. Dans l'exemple particulier illustré aux figures 2b et 2c, une première tension radiofréquence peut être appliquée pour éjecter les ions de première génération résiduels G1', puis une deuxième tension radiofréquence peut être appliquée pour éjecter le deuxième type d'ions de deuxième génération G2T2. Alternativement, les première et deuxième tensions radiofréquences peuvent être appliquées simultanément.

Les figures 1a et 2c montrent une étape ii), qui est référencée 150 en figure 1a, selon laquelle les ions de deuxième génération du premier type G2T1 sélectionnés et piégés dans le piège ionique sont refroidis, au moyen du module de refroidissement Re précédemment décrit.

Les figures 1a, 2c et 2d montrent une étape iii), qui est référencée 160 en figure 1a, selon laquelle les ions de deuxième génération du premier type G2T1 refroidis sont photo-fragmentés au moyen du laser de photo-fragmentation L émettant à une deuxième longueur d'onde À2, pour l'obtention d'une pluralité d'ions de troisième génération G3. La pluralité d'ions de troisième génération G3 est différente de la pluralité d'ions de deuxième génération, et la pluralité d'ions de troisième génération G3 est au moins d'un premier type. La deuxième longueur d'onde λ2 est choisie en fonction des ions de deuxième génération du premier type G2T1 à photo-fragmenter. La deuxième longueur d'onde λ2 est typiquement différente de la première longueur d'onde λ1.

Dans le cas qui vient d'être décrit, le laser de photo-fragmentation L émet à la première longueur d'onde λ1 pour la première photo-fragmentation de l'étape 130, et émet à la deuxième longueur d'onde λ2 pour la deuxième photo-fragmentation de l'étape 160. Alternativement, deux lasers de photo-fragmentation peuvent être utilisés : un premier laser de photo-fragmentation émettant à la première longueur d'onde λ1 pour la première photo-fragmentation de l'étape 130, et un deuxième laser de photo-fragmentation émettant à la deuxième longueur d'onde λ2 pour la deuxième photo-fragmentation de l'étape 160.

Les figures 1a et 2d montrent une étape 170 selon laquelle la pluralité d'ions de dernière génération, en l'occurrence la pluralité d'ions de troisième génération G3, est détectée au moyen d'un détecteur De. La détection de la pluralité d'ions de dernière génération peut par exemple être réalisée au moyen d'un channeltron CPM (de l'anglais « channel photomultiplier »), d'une galette de microcanaux MCP (de l'anglais « multichannel plate ») ou d'un détecteur de Daly. On obtient ainsi la signature spectroscopique du premier type G2T1 d'ions de deuxième génération.

Dans l'exemple particulier qui vient d'être décrit, un premier type G2T1 d'ions de deuxième génération et un deuxième type G2T2 d'ions de deuxième génération sont obtenus à l'issue de la photo-fragmentation des ions G1 de première génération. Après avoir obtenu la signature spectroscopique du premier type G2T1 d'ions de deuxième génération en réalisant un premier cycle des étapes 110 à 170, il est alors possible d'obtenir la signature spectroscopique du deuxième type G2T2 d'ions de deuxième génération en réalisant un deuxième cycle 100', représenté à la figure 1a', comportant :
- l'étape 110 selon laquelle la pluralité G1 d'ions de première génération est piégée dans le piège ionique P ;
- l'étape 120 selon laquelle la pluralité G1 d'ions de première génération piégée dans le piège ionique P est refroidie ;
- l'étape 130 selon laquelle la pluralité G1 d'ions de première génération refroidis est photo-fragmentée pour l'obtention, dans l'exemple particulier considéré, du premier type G2T1 d'ions de deuxième génération et du deuxième type G2T2 d'ions de deuxième génération ;
- une étape 140' selon laquelle le deuxième type G2T2 d'ions de deuxième génération est sélectionné dans le piège ionique P en éjectant hors du piège ionique tout ions de première génération résiduel et tout ion de deuxième génération d'un type différent du deuxième type ;
- une étape 150' selon laquelle les ions de deuxième génération du deuxième type sélectionnés et piégés dans le piège ionique P sont refroidis ;
- une étape 160' selon laquelle les ions de deuxième génération du deuxième type refroidis sont photo-fragmentés pour l'obtention d'une deuxième pluralité d'ions de troisième génération G3' ;
- une étape 170' selon laquelle la deuxième pluralité d'ions de troisième génération G3' est détectée.
On obtient ainsi la signature spectroscopie du deuxième type G2T2 d'ions de deuxième génération.

De même que décrit pour l'étape 140, le spectromètre de masse Sp est utilisé lors de l'étape 140'. Quel que soit le contenu du piège ionique P avant l'étape 140' de sélection, on garantit que le piège ionique P ne contient sensiblement plus que le deuxième type d'ions de deuxième génération G2T2 à l'issue de ladite étape 140' de sélection. On entend par « sensiblement » le fait qu'une petite quantité résiduelle ε des ions à éjecter peut subsister au sein du piège ionique P après l'étape 140 de sélection, ainsi que précédemment décrit pour l'étape 140.

De même que décrit pour l'étape 150, le module de refroidissement Re est utilisé lors de l'étape 150'.

Le laser de photo-fragmentation L émettant à une troisième longueur d'onde λ2' est utilisé lors de l'étape 160'. La troisième longueur d'onde λ2' est choisie en fonction des ions de deuxième génération du deuxième type G2T2 à photo-fragmenter. La troisième longueur d'onde λ2' est typiquement différente de la première longueur d'onde λ1 et de la deuxième longueur d'onde λ2.

Dans le cas qui vient d'être décrit, le laser de photo-fragmentation L émet à la première longueur d'onde λ1 pour la première photo-fragmentation de l'étape 130, émet à la deuxième longueur d'onde λ2 pour la deuxième photo-fragmentation de l'étape 160 et émet à la troisième longueur d'onde λ2' pour la troisième photo-fragmentation de l'étape 160'. Alternativement, deux lasers de photo-fragmentation peuvent être utilisés : un premier laser de photo-fragmentation émettant à la première longueur d'onde λ1 pour la première photo-fragmentation de l'étape 130, et un deuxième laser de photo-fragmentation émettant à la deuxième longueur d'onde λ2 pour la deuxième photo-fragmentation de l'étape 160 et émettant à la troisième longueur d'onde λ2' pour la troisième photo-fragmentation de l'étape 160'. Selon une autre alternative, trois lasers de photo-fragmentation peuvent être utilisés : un premier laser de photo-fragmentation émettant à la première longueur d'onde λ1 pour la première photo-fragmentation de l'étape 130, un deuxième laser de photo-fragmentation émettant à la deuxième longueur d'onde λ2 pour la deuxième photo-fragmentation de l'étape 160 et un troisième laser de photo-fragmentation émettant à la troisième longueur d'onde λ2' pour la troisième photo-fragmentation de l'étape 160'.

De même que décrit pour l'étape 170, le détecteur De est utilisé lors de l'étape 170'.

D'une manière générale, il peut avantageusement être réalisé autant de cycles que de types différents d'ions de deuxième génération, de manière à obtenir la signature spectroscopique de chaque type d'ion de deuxième génération.

La figure 1b montre un diagramme des étapes d'un procédé 200 de caractérisation d'ions selon un deuxième mode de réalisation de l'invention. Dans le deuxième mode de réalisation de l'invention, les étapes i), ii) et iii), qui sont respectivement référencées 140, 150 et 160 sur les figures 1a et 1b, sont réalisées séquentiellement N fois, N étant un entier naturel supérieur ou égal à 2. Le numéro de chaque génération est incrémenté de 1 à chaque réalisation de la dite séquence.

Nous décrivons à présent un exemple de réalisation du procédé 200 selon le deuxième mode de réalisation, selon lequel le nombre N de réalisations de la séquence des étapes i), ii) et iii) est égal à 2.

Le premier enchaînement des étapes 110, 120, 130, 140, 150 et 160 du procédé 200 selon le deuxième mode de réalisation est identique à l'enchaînement des étapes 110, 120, 130, 140, 150 et 160 du procédé 100 selon le premier mode de réalisation, qui a été précédemment décrit. En effet, pour N = 1, le procédé 200 selon le deuxième mode de réalisation est identique au procédé 100 selon le premier mode de réalisation. A ce stade, la séquence des étapes i), ii) et iii) a donc été réalisée une fois. Pour N = 2, la séquence des étapes i), ii) et iii) est ensuite réalisée une deuxième fois :
- selon la deuxième étape i), le premier type d'ion de troisième génération est sélectionné dans le piège ionique en éjectant hors du piège ionique tout ion de deuxième génération résiduel et tout ion de troisième génération d'un type différent du premier type ;
- selon la deuxième étape ii), les ions de troisième génération du premier type sélectionnés et piégés dans le piège ionique sont refroidis ;
- selon la deuxième étape iii), les ions de troisième génération du premier type refroidis sont photo-fragmentés pour l'obtention d'une pluralité d'ions de quatrième génération, la pluralité d'ions de quatrième génération étant différente de la pluralité d'ions de troisième génération, la pluralité d'ions de quatrième génération étant au moins d'un premier type.

Enfin, le procédé 200 selon le deuxième mode de réalisation de l'invention comporte l'étape 170 selon laquelle la pluralité d'ions de dernière génération, en l'occurrence la pluralité d'ions de quatrième génération, est détectée. On obtient ainsi la signature spectroscopique du premier type d'ions de troisième génération. D'une manière générale, la signature spectroscopique de chaque type d'ion de troisième génération est avantageusement déterminée.

## Revendications

1. Procédé (100, 200) de caractérisation d'ions comportant :
- une étape (110) selon laquelle une pluralité (G1) d'ions de première génération est piégée dans un piège ionique (P) ;
- une étape (120) selon laquelle la pluralité (G1) d'ions de première génération piégée dans le piège ionique est refroidie ;
- une étape (130) selon laquelle la pluralité (G1) d'ions de première génération refroidis est photo-fragmentée pour l'obtention d'une pluralité (G2T1, G2T2) d'ions de deuxième génération, la pluralité d'ions de deuxième génération étant différente de la pluralité d'ions de première génération, la pluralité d'ions de deuxième génération étant au moins d'un premier type (G2T1) ;
- une étape (140) i) selon laquelle le premier type d'ions de deuxième génération (G2T1) est sélectionné dans le piège ionique en éjectant hors du piège ionique tout ion de première génération résiduel et tout ion de deuxième génération d'un type différent du premier type ;
- une étape (150) ii) selon laquelle les ions de deuxième génération du premier type sélectionnés et piégés dans le piège ionique sont refroidis ;
- une étape (160) iii) selon laquelle les ions de deuxième génération du premier type refroidis sont photo-fragmentés pour l'obtention d'une pluralité d'ions de troisième génération (G3), la pluralité d'ions de troisième génération étant différente de la pluralité d'ions de deuxième génération, la pluralité d'ions de troisième génération étant au moins d'un premier type ;
- une étape (170) selon laquelle la pluralité d'ions de dernière génération (G3) est détectée.

2. Procédé (100, 200) de caractérisation selon la revendication précédente **caractérisé en ce qu'**il comporte une étape selon laquelle la pluralité d'ions de première génération est sélectionnée par une technique de spectrométrie de masse.

3. Procédé (100, 200) de caractérisation selon l'une quelconque des revendications précédentes **caractérisé en ce que** l'étape (170) selon laquelle la pluralité d'ions de dernière génération (G3) est détectée est réalisée par une technique de spectrométrie de masse.

4. Procédé (100, 200) de caractérisation selon l'une quelconque des revendications précédentes **caractérisé en ce que** les étapes i), ii) et iii) (140, 150, 160) forment une séquence qui est réalisée N fois, N étant un entier naturel supérieur ou égal à 2, le numéro de chaque génération étant incrémenté de 1 à chaque réalisation de ladite séquence.

## Patentansprüche

1. Verfahren (100, 200) zur Charakterisierung von Ionen, aufweisend:
- einen Schritt (110), gemäß dem eine Vielzahl (G1) von Ionen erster Generation in einer Ionenfalle (P) gefangen wird;
- einen Schritt (120), gemäß dem die Vielzahl (G1) von Ionen erster, in der Ionenfalle gefangener Generation gekühlt wird;
- einen Schritt (130), gemäß dem die Vielzahl (G1) gekühlter Ionen erster Generation für den Erhalt einer Vielzahl (G2T1, G2T2) von Ionen zweiter Generation photofragmentiert wird, wobei die Vielzahl von Ionen zweiter Generation von der Vielzahl von Ionen erster Generation unterschiedlich ist, wobei die Vielzahl von Ionen zweiter Generation mindestens eines ersten Typs (G2T1) ist;
- einen Schritt (140) i), gemäß dem der erste Typ von Ionen zweiter Generation (G2T1) in der lonenfalle durch Auswerfen aus der lonenfalle jedes verbliebenen Ions erster Generation und jedes Ions zweiter Generation eines Typs, der sich vom ersten Typ unterscheidet, ausgewählt wird;
- einen Schritt (150) ii), gemäß dem die in der Ionenfalle ausgewählten und gefangenen Ionen zweiter Generation des ersten Typs gekühlt werden;
- einen Schritt (160) iii), gemäß dem die gekühlten Ionen zweiter Generation des ersten Typs für den Erhalt einer Vielzahl von Ionen dritter Generation (G3) photofragmentiert werden, wobei sich die Vielzahl von Ionen dritter Generation von der Vielzahl von Ionen zweiter Generation unterscheidet, wobei die Vielzahl von Ionen dritter Generation mindestens eines ersten Typs ist;
- einen Schritt (170), gemäß dem die Vielzahl von Ionen letzter Generation (G3) ermittelt wird.

2. Charakterisierungsverfahren (100, 200) nach vorangehendem Anspruch, **dadurch gekennzeichnet, dass** es einen Schritt aufweist, gemäß dem die Vielzahl von Ionen erster Generation durch eine Massenspektrometrietechnik ausgewählt wird.

3. Charakterisierungsverfahren (100, 200) nach einem der vorangehenden Ansprüche, **dadurch gekennzeichnet, dass** der Schritt (170), gemäß dem die Vielzahl von Ionen letzter Generation (G3) ermittelt wird, durch eine Massenspektrometrietechnik durchgeführt wird.

4. Charakterisierungsverfahren (100, 200) nach einem der vorangehenden Ansprüche, **dadurch gekennzeichnet, dass** die Schritte i), ii) und iii) (140, 150, 160) eine Sequenz bilden, die N Mal durchgeführt wird, wobei N eine natürliche Ganzzahl größer oder gleich 2 ist, wobei die Nummer jeder Generation bei jeder Durchführung der Sequenz um 1 inkrementiert wird.

## Claims

1. Method (100, 200) for characterising ions comprising:
- a step (110) according to which a plurality (G1) of first-generation ions is trapped in an ion trap (P);
- a step (120) according to which the plurality (G1) of first-generation ions trapped in an ion trap is cooled;
- a step (130) according to which the plurality (G1) of cooled first-generation ions is photo-fragmented in order to obtain a plurality (G2T1, G2T2) of second-generation ions, the plurality of second-generation ions being different from the plurality of first-generation ions, the plurality of second-generation ions being at least of one first type (G2T1);
- a step (140) i) according to which the first type of second-generation ions (G2T1) is selected in the ion trap by ejecting, out of the ion trap, any residual first-generation ion and any second-generation ion of a type different from the first type;
- a step (150) ii) according to which the second-generation ions of the first type selected and trapped in the ion trap are cooled;
- a step (160) iii) according to which the cooled second-generation ions of the first type are photo-fragmented to obtain a plurality of third-generation ions (G3), the plurality of third-generation ions being different from the plurality of second-generation ions, the plurality of third-generation ions being at least of one first type;
- a step (170) according to which the plurality of last-generation ions (G3) is detected.

2. Method (100, 200) for characterising according to the preceding claim **characterised in that** it comprises a step according to which the plurality of first-generation ions is selected using a mass spectrometry technique.

3. Method (100, 200) for characterising according to any preceding claim **characterised in that** the step (170) according to which the plurality of last-generation ions (G3) is detected and realised by a mass spectrometry technique.

4. Method (100, 200) for characterising according to any preceding claim **characterised in that** the steps i), ii) and iii) (140, 150, 160) form a sequence that is carried out N times, N being a natural number greater than or equal to 2, with the number of each generation being incremented by 1 at each carrying out of said sequence.
